(19) Europäisches Patentamt
European Patent Office
Office européen des brevets

(11) Veröffentlichungsnummer: **0 033 885**
**B1**

(12) # EUROPÄISCHE PATENTSCHRIFT

(45) Veröffentlichungstag der Patentschrift: **21.03.84**

(51) Int. Cl.³: **C 07 C 41/16**, C 07 C 43/29

(21) Anmeldenummer: **81100540.4**

(22) Anmeldetag: **26.01.81**

(54) **Verfahren zur Herstellung von (2-Chlor-4-trifluormethyl-phenyl)-(3-methoxy-phenyl)-ether.**

(30) Priorität: **08.02.80 DE 3004695**

(43) Veröffentlichungstag der Anmeldung: **19.08.81 Patentblatt 81/33**

(45) Bekanntmachung des Hinweises auf die Patenterteilung: **21.03.84 Patentblatt 84/12**

(84) Benannte Vertragsstaaten: **BE DE IT**

(56) Entgegenhaltungen:
**EP-A- 0 000 351**
**EP-A- 0 000 359**
**EP-A- 0 003 562**
**DE-A- 2 333 848**
**DE-B- 2 304 006**

(73) Patentinhaber: **BAYER AG**
**Konzernverwaltung RP Patentabteilung**
**D-5090 Leverkusen 1 Bayerwerk (DE)**

(72) Erfinder: **Förster, Heinz, Dr.**
**Am Eckbusch 47**
**D-5600 Wuppertal 1 (DE)**
Erfinder: **Priesnitz, Uwe, Dr.**
**Severinstrasse 58**
**D-5650 Solingen 1 (DE)**

**EP 0 033 885 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von (2-Chlor-4-trifluormethyl-phenyl)-(3-methoxy-phenyl)-ether

Die Erfindung betrifft ein neues Verfahren zur herstellung des bekannten (2-Chlor-4-trifluormethyl-phenyl)-(3-methoxy-phenyl)-ethers, der als Zwischen-produkt zur Synthese von Herbiziden verwendet werden kann.

Es ist bereits bekannt, daß man bestimmte Trifluormethylphenyl-phenyl-ether, wie z. B. (2-Chlor-4-trifluormethyl-phenyl)-(3-methoxy-phenyl)-ether erhält, wenn man 4-Trifluormethyl-1-halogen-benzole, wie z. B. 1,2-Dichlor-4-trifluormethyl-benzol, mit Alkaliphenolaten, wie z. B. Natrium-3-methoxy-phenolat, in polaren Lösungsmitteln, wie z. B. Dimethylsulfoxid, längere Zeit auf ungefähr 140 °C erhitzt (vgl. DE-OS 2 333 848). Nach diesem Verfahren erhält man jedoch stark verunreinigte Produkte in unbefriedigenden Ausbeuten. Das meistens bei diesem Verfahren verwendete Lösungsmittel Dimethylsulfoxid zersetzt sich zudem exotherm bei Temperaturen oberhalb 130 °C ; daher ist das bekannte Verfahren für die Durchführung im großtechnischen maßstab wenig geeignet.

In der DE-AS 2 304 006 wird der (2-Chlor-4-trifluormethyl-phenyl)-(3-methoxy-phenyl)-ether in dem Herstellungsbeispiel 5 als Ausgangsmaterial erwähnt (vgl. Spalte 6, Zeilen 45 und 46). Es sind aber keine speziellen Hinweise zur Synthese dieses Stoffes vorhanden. Außerdem geht aus den Angaben hervor, die in der DE-AS 2 304 006 in Spalte 4 (Zeilen 33 bis 68) und Spalte 5 (Zeilen 1 bis 6) enthalten sind, daß das dort allgemein beschriebene Verfahren zur Herstellung von Diphenylethern bevorzugt in Gegenwart eines Kupferkatalysators bei Temperaturen zwischen 130 und 200 °C, insbesondere zwischen 140 und 180 °C, durchgeführt wird. Es finden sich keine Anhaltspunkte dafür, daß die betreffende Umsetzung auch bei tieferen Temperaturen und zudem noch in Abwesenheit eines Katalysators, zufriedenstellend abläuft.

Die EP-OS 0 003 562 betrifft ein Verfahren zur Herstellung von Hydroxy-diphenylethern durch Umsetzung von Halogen-Verbindungen mit Di-Hydroxybenzolen in Gegenwart von Calciumhydroxid. Die dabei als Reaktionskomponenten eingesetzten Benzol-Derivate enthalten also jeweils zwei Hydroxygruppen. Entsprechende Umsetzungen des Resorcin-mono-methylethers, in dem nur eine Hydroxygruppe vorhanden ist, werden jedoch nicht offenbart.

Es wurde nun gefunden, daß man (2-Chlor-4-trifluormethyl-phenyl)-(3-methoxy-phenyl)-ether der Formel

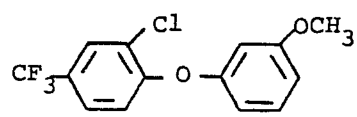

(I)

erhält, wenn man 1,2-Dichlor-4-trifluormethyl-benzol der Formel

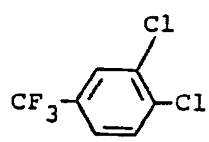

(II)

mit Resorcin-mono-methylether der Formel

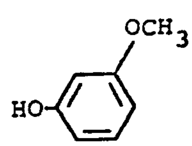

(III)

und basischen Alkali- oder Erdalkaliverbindungen bzw. mit den aus dem Resorcin-mono-methylether der Formel (III) und den basischen Alkali- oder Erdalkaliverbindungen gebildeten Salzen in Gegenwart von Verdünnungs-mitteln bei Temperaturen zwischen 80 und 110 °C umsetzt.

Der Verlauf des erfindungsgemäßen Verfahrens konnte unter Berücksichtigung des bekannten Standes der Technik nicht erwartet werden. So wird in der DE-OS 23 33 848 erwähnt, daß das dort als (a) bezeichnete Verfahren bei Temperaturen zwischen vorzugsweise 80 und 160 °C durchgeführt wird. Im Falle des Beispiels 1 liegt die Reaktionstemperatur bei 140 °C. Die Ausbeute an Produkt beträgt nur 66 %. — Weiterhin wird auch in der DE-OS 23 04 006 ausdrücklich darauf hingewiesen, daß die zur Diskussion stehende Diphenylether-Synthese insbesondere bei Temperaturen zwischen 140 und 180 °C abläuft (vgl. Spalte 5, Zeilen 5 und 6). — Ferner ist dem in der EP-OS 0 003 562 enthaltenen Beispiel 1 zu entnehmen, daß zur Herstellung des betreffenden Produktes eine Reaktionstemperatur von 130 °C und eine Umsetzungsdauer von 8 Stunden erforderlich ist.

Basierend auf diesen Angaben mußte davon ausgegangen werden, daß die Herstellung von (2-Chlor-4-trifluormethyl-phenyl)-3-methoxy-phenyl)-ether nur dann mit befriedigender Ausbeute gelingt, wenn

man bei so hohen Reaktionstemperaturen arbeitet wie bei den vergleichbaren vorbekannten Umsetzungen. Im Gegensatz zu den Erwartungen läuft das erfindungsgemäße Verfahren aber bereits bei deutlich niedrigeren Reaktionstemperaturen innerhalb relativ kurzer Zeit ab und liefert das gewünschte Produkt in hoher Ausbeute und Reinheit. Dieser Befund ist im Hinblick auf den bekannten Stand der Technik als äußerst überraschend zu bezeichnen.

Das erfindungsgemäße Verfahren weist verschiedene Vorteile auf. So wird durch die Durchführung bei relativ niedriger Temperatur unnötiger Energieaufwand vermieden ; die Gefährdung der Umwelt wird vermindert ; die Bildung von unerwünschten Nebenprodukten wird stark eingeschränkt und die Ausbeute an der Verbindung der Formel (I) entsprechend erhöht.

Der Verlauf des erfindungsgemäßen Verfahrens kann durch das folgende Formelschema wiedergegeben werden :

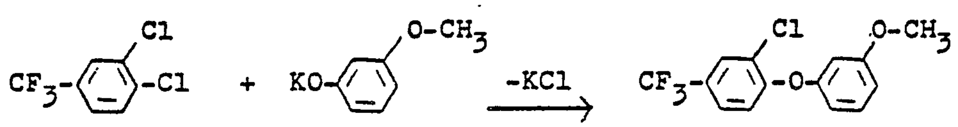


Die Ausgangsstoffe der Formeln (II) und (III) sind bekannt.

Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone, wie Aceton, Methylethyl-, Methyl-isopropyl- und Methylisobutylketon, Ester, wie Essigsäuremethyl-ester und -ethylester, Nitrile, wie z. B. Acetonitril und Propionitril, Amide, wie z. B. Dimethylformamid, Dimethylacetamid und N-Methyl-pyrrolidon, sowie Dimethylsulfoxid, Tetramethylensulfon und Hexamethylphosphorsäuretriamid.

Vorzugsweise werden von den genannten Verdünnungsmitteln die aprotisch polaren Solventien, insbesondere Dimethylsulfoxid, und gegebenenfalls zusätzlich weitere Lösungsmittel, wie z. B. Toluol oder Xylole verwendet.

Basische Alkali- oder Erdalkaliverbindungen, welche beim erfindungsgemäßen Verfahren eingesetzt werden können, sind beispielsweise Alkali- oder Erdalkalioxide, -hydroxide, -carbonate, -hydride, -amide und -alkoholate.

Als Beispiele für Verbindungen aus dieser Reihe seien Natrium- und Kalium-hydroxid, Calcium-oxid und -hydroxid, Kaliumcarbonat, Natrium- und Calcium-hydrid, Natriumamid sowie Natriumethylat und Kalium-tert.-butylat genannt. Natrium- und Kaliumhydroxid werden vorzugsweise verwendet.

Die Reaktionstemperatur wird bei der Durchführung des erfindungsgemäßen Verfahrens zwischen 80 und 110 °C gehalten. Im allgemeinen wird bei Normaldruck gearbeitet. Es ist jedoch auch möglich, das Verfahren bei geringfügig erhöhtem oder vermindertem Druck, etwa zwischen 0,1 und 10 bar, durchzuführen.

Auf 1 Mol 1,2-Dichlor-4-Trifluormethylbenzol der Formel (II) werden 1 bis 1,5 Mol, vorzugsweise 1,1 bis 1,4 Mol Resorcin-mono-methylether der Formel (III) und 1 bis 2 Mol, vorzugsweise 1,1 bis 1,5 Mol einer basischen Alkaliverbindung oder entsprechende Moläquivalente einer basischen Erdalkaliverbindung eingesetzt.

Im allgemeinen arbeitet man bei dem erfindungsgemäßen Verfahren in der Weise, daß man Resorcin-mono-methylether der Formel (III) zusammen mit einer basischen Alkali- oder Erdalkali-Verbindung in einem Solvens vorlegt, gegebenenfalls entstehendes Wasser azeotrop entfernt, dann 1,2-Dichlor-4-trifluormethylbenzol der Formel (II) in Substanz oder in Lösung bei Raumtemperatur hinzugibt und anschließend das Reaktionsgemisch langsam auf die gewünschte Reaktionstemperatur erhitzt. Es ist aber auch möglich, sämtliche Reaktionspartner bei Raumtemperatur, d. h. zwischen 10 °C und 30 °C, im Solvens zu lösen oder zu dispergieren und danach die komplette Mischung auf die Reaktionstemperatur zu bringen.

Die Aufarbeitung erfolgt nach üblichen Methoden. Im allgemeinen verfährt man so, daß man das Reaktionsgemisch nach beendeter Umsetzung einengt, den Rückstand mit Wasser und einem in Wasser wenig löslichen organischen Lösungsmittel versetzt, die organische Phase abtrennt, wäscht, trocknet und einengt.

In einer bevorzugten Ausführungsform des erfindungsgemäßen Verfahrens wird zunächst Resorcin-mono-methyl-ether der Formel (III) mit einer basischen Alkali- oder Erdalkaliverbindung, vorzugsweise in Gegenwart eines Verdünnungsmittels, welches mit Wasser ein azeotrop siedendes Gemisch bildet, wie z. B. Toluol, zum Sieden erhitzt und das bei der Reaktion gebildete Wasser wird über einen Wasserabscheider ausgekreist. Dann wird mit einem der oben angegebenen polaren Lösungsmittel verdünnt und 1,2-Dichlor-4-trifluormethyl-benzol der Formel (II) dazugegeben. Das Reaktionsgemisch wird einige Stunden, vorzugsweise 3 bis 5 Stunden bei der oben angegebenen Reaktionstemperatur gerührt. Zur Aufarbeitung wird das Lösungsmittel im Vakuum abdestilliert, der Rückstand mit Wasser und einem mit Wasser nicht mischbaren organischen Lösungsmittel, wie z. B. Cyclohexan, verrührt. Dann wird über

Kieselgel abgesaugt. Die organische Phase wird mit verdünnter Natronlauge und dann mit Wasser gewaschen, getrocknet und filtriert. Vom Filtrat wird das Lösungsmittel unter vermindertem Druck sorgfältig abdestilliert, wobei das Produkt als öliger Rückstand verbleibt.

Der nach dem erfindungsgemäßen Verfahren herstellbare (2-Chlor-4-trifluormethyl-phenyl)-(3-methoxy-phenyl)-ether der Formel (I) ist ein wertvoller Ausgangsstoff zur Synthese von substituierten Diphenylethern, welche hervorragende herbizide Eigenschaften besitzen (vgl. DE-OS 23 33 848).

So läßt sich beispielsweise der 2-Chlor-4'-nitro-4-trifluormethyl-3'-methoxy-diphenylether der Formel

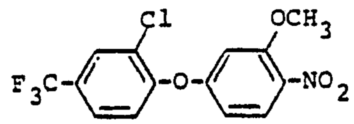

herstellen, indem man (2-Chlor-4-trifluormethyl-phenyl)-(3-methoxy-phenyl)-ether mit Salpetersäure in Gegenwart von Eisessig und Acetanhydrid umsetzt. Diese Synthese läßt sich formelmäßig wie folgt veranschaulichen :

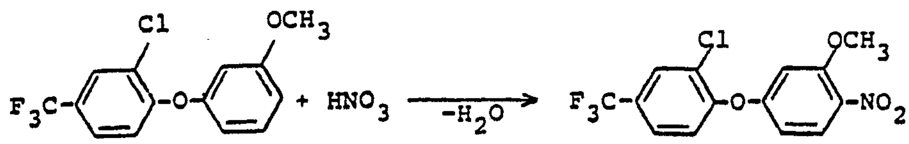

Das erfindungsgemäße Verfahren wird durch die nachfolgenden Beispiele veranschaulicht.

Beispiel 1

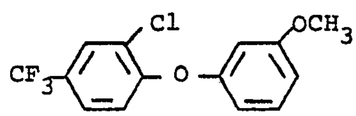

158 g (1,285 Mol) Resorcinmonomethylether werden zusammen mit 500 ml Toluol und 82,4 g Kaliumhydroxidpulver unter Rückfluß erhitzt. Das Wasser wird azeotrop ausgekreist. Zum zurückbleibenden Salz werden 300 ml Dimethylformamid und 215 g (1,0 Mol) 1,2-Dichlor-4-trifluormethyl-benzol zugesetzt. Das Reaktionsgemisch wird langsam auf 90 °C erhitzt. Die Reaktionsmischung wird dann 4 Stunden bei 90-100 °C gehalten. Das Lösungsmittel wird im Vakuum abdestilliert. Dann werden 100 ml Wasser und 500 ml Cyclohexan zugesetzt und der Ansatz gut verrührt. Dann wird über Kieselgur abgesaugt und die Cyclohexanphase einmal mit 1 n wäßriger Natronlauge und dann mit Wasser neutral gewaschen. Die Lösung wird getrocknet und im Vakuum eingeengt.

Man erhält als Rückstand (2-Chlor-4-trifluormethyl-phenyl)-(3-methoxy-phenyl)-ether in Ausbeuten zwischen 80 und 90 % der Theorie. Siedepunkt : 121-125 °C/1-2 mbar

$22_{n_D}$ = 1,533 1.

Herstellung von 2-Chlor-4'-nitro-4-trifluormethyl-3'-methoxy-diphenylether ausgehend von (2-Chlor-4-trifluormethyl-phenyl)-(3-methoxy-phenyl)-ether

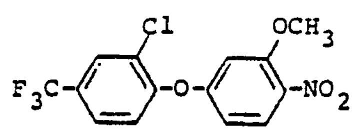

Eine Lösung von 8,35 ml (0,2 Mol) Salpetersäure (Dichte 1,5) in 30 ml Essigsäure wird zu einer Lösung von 60,5 g (0,2 Mol) (2-Chlor-4-trifluormethyl-phenyl)-(3-methoxy-phenyl)-ether in 40 ml Acetanhydrid bei 25 bis 30 °C innerhalb von 30 Minuten tropfenweise gegeben. Das Reaktionsgemisch wird 4 Stunden bei 25 bis 30 °C gerührt und dann auf Eis gegossen. Man extrahiert mit Methylenchlorid, wäscht die Extraktionslösung mit Wasser, mit verdünnter Natronlauge und wieder mit Wasser.

Nach Abziehen des Lösungsmittels wird das zurückbleibende Produkt durch Umkristallisieren aus Methanol gereinigt.

Man erhält 36 g (51 % der Theorie) 2-Chlor-4'-nitro-4-trifluormethyl-3'-methoxy-diphenylether vom Schmelzpunkt 103 °C.

**Ansprüche**

1. Verfahren zur Herstellung von (2-Chlor-4-trifluormethyl-phenyl)-(3-methoxy-phenyl)-ether der Formel

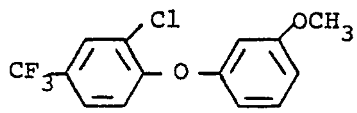

(I)

dadurch gekennzeichnet, daß man 1,2-Dichlor-4-trifluormethyl-benzol der Formel

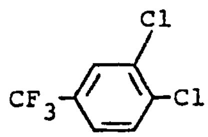

(II)

mit Resorcin-mono-methylether der Formel

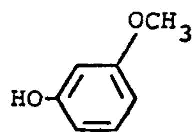

(III)

und basischen Alkali- oder Erdalkaliverbindungen bzw. mit den aus dem Resorcin-mono-methylether der Formel (III) und den basischen Alkali- oder Erdalkaliverbindungen gebildeten Salzen in Gegenwart von Verdünnungsmitteln bei Temperaturen zwischen 80 und 110 °C umsetzt.

2. Verfahren gemäß Anspruch 1, dadurch gekennzeichnet, daß man als basische Alkaliverbindungen Natriumhydroxid, Kaliumhydroxid, Kaliumcarbonat, Natriumhydrid, Natriumamid, Natriumethylat oder Kalium-tert.-butylat einsetzt.

3. Verfahren gemäß Anspruch 1, dadurch gekennzeichnet, daß man als basische Erdalkaliverbindung Calciumoxid, Calciumhydroxid oder Calciumhydrid einsetzt.

4. Verfahren gemäß Anspruch 1, dadurch gekennzeichnet, daß man als Verdünnungsmittel Dimethylsulfoxid und Toluol oder Xylole einsetzt.

5. Verfahren gemäß Anspruch 1, dadurch gekennzeichnet, daß man auf 1 Mol an 1,2-Dichlor-4-trifluormethyl-benzol der Formel (II) 1 bis 1,5 Mol an Resorcin-mono-methylether der Formel (III) sowie 1 bis 2 Mol an einer basischen Alkaliverbindung bzw. entsprechende Moläquivalente an einer basischen Erdalkaliverbindung einsetzt.

**Claims**

1. Process for the preparation of (2-chloro-4-trifluoromethyl-phenyl)-(3-methoxy-phenyl) ether of the formula

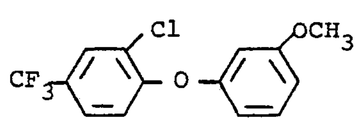

(I)

characterised in that 1,2-dichloro-4-trifluoromethylbenzene of the formula

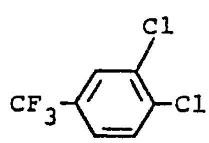

(II)

is reacted with resorcinol monomethyl ether of the formula

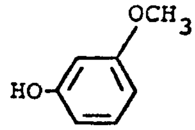

(III)

and basic alkali metal or alkaline earth metal compounds or with the salts formed from resorcinol monomethyl ether of the formula (III) and the basic alkali metal or alkaline earth metal compounds in the presence of diluents at temperatures between 80 and 110 °C.

2. Process according to Claim 1, characterised in that the basic alkali metal compounds used are sodium hydroxide, potassium hydroxide, potassium carbonate, sodium hydride, sodium amide, sodium methylate or potassium tert.-butylate.

3. Process according to Claim 1, characterised in that the basic alkaline earth metal compound used is calcium oxide, calcium hydroxide or calcium hydride.

4. Process according to claim 1, characterised in that dimethyl sulphoxide and toluene or xylenes are used as the diluents.

5. Process according to Claim 1, characterised in that per mole of 1,2-dichloro-4-trifluoromethylbenzene of the formula (II) 1 to 1.5 moles of resorcinol monomethyl ether of the formula (III) and 1 to 2 moles of a basic alkali metal compound or corresponding molar equivalents of a basic alkaline earth metal compound are used.

**Revendications**

1. Procédé de préparation de (2-chlore-4-trifluorométhyle-phényle)-(3-methoxy-phényle)-éther de formule

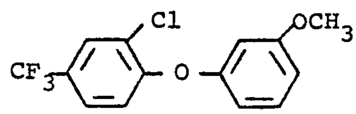

(I)

caractérisé en ce qu'on fait réagir 1,2-dichlore-4-trifluorométhyl-benzène de formule

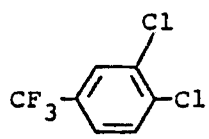

(II)

avec l'éther de résorcine monométhylique de formule

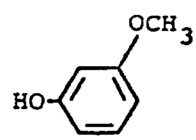

(III)

et des composés basics d'alcali ou d'alcali de terre respectivement avec les sels, lesquels sont formés de l'éther de résorcine monométhylique de formule (III) et les composés basics d'alcali ou d'alcali de terre, en présence des diluants à des températures de 80 et 110 °C.

2. Procédé selon la revendication 1, caractérisé en ce qu'on utilise comme des composés basics d'alcali d'hydroxyde de sodium, d'hydroxyde de potassium, de carbonate neutre de potassium, d'hydrure de sodium, d'amidure de sodium, d'éthylate de soude ou de tert.-butylate de soude.

3. Procédé selon la revendication 1, caractérisé en ce qu'on utilise comme de composé d'alcali de terre d'oxide de calcium, d'hydroxyde de calcium ou d'hydrure de calcium.

4. Procédé selon la revendication 1, caractérisé en ce qu'on utilise comme des diluants de diméthylsulfoxyde et de toluène ou de xylène.

5. Procédé selon la revendication 1, caractérisé en ce que, pour 1 mole de 1,2-dichlore-4-trifluorométhyl-benzène de formule (II), on utilise de 1 à 1,5 mole d'éther de resorcine monométhylique respectivement des équivalents molaires correspondants d'une composé d'alcaline de terre.